# EUROPEAN PATENT APPLICATION

(11) **EP 4 310 176 A1**
(43) Date of publication of application: **24.01.2024**
(21) Application number: 22771459.9
(22) Date of filing: 16.03.2022
(51) Int. Cl.: C12N 5/071, A61K 35/44, A61P 9/10, C12N 5/10, C12N 15/12

(54) **PERICYTE HAVING BASIC FIBROBLAST GROWTH FACTOR (BFGF) GENE INTRODUCED THEREIN**

(30) Priority: 17.03.2021 JP 2021043100
(71) Applicant: Astellas Pharma Inc., Chuo-ku Tokyo 103-8411 (JP); OSAKA UNIVERSITY, Suita-shi Osaka 565-0871 (JP)
(72) Inventor: SHIMATANI, Kenichiro, Tokyo 103-8411 (JP); SATO, Hiromu, Tokyo 103-8411 (JP); SASAI, Masao, Suita-shi, Osaka 565-0871 (JP); SAWA, Yoshiki, Suita-shi, Osaka 565-0871 (JP); SAITO, Atsuhiro, Suita-shi, Osaka 565-0871 (JP); MIYAGAWA, Shigeru, Suita-shi, Osaka 565-0871 (JP)
(74) Representative: Hoffmann Eitle
(86) International application number: PCT/JP2022/011788
(87) International publication number: WO 2022/196714

(57) **Abstract**

An object of the present invention is to provide a cell therapy that is expected to be useful as an angiogenic therapy for a peripheral vascular disease such as critical lower limb ischemia. Specifically, the object is to provide a pericyte having high angiogenic potential and a method for producing the same. A pericyte introduced a bFGF gene and a method for producing the same are provided.

## Description

### TECHNICAL FIELD

The present invention relates to a pericyte introduced a basic fibroblast growth factor (bFGF) gene, a pharmaceutical composition including the pericyte, a method for producing the pericyte, an angiogenic therapy including administering the pericyte, and the like.

### BACKGROUND ART

A capillary connects an arteriole and a venule, and is distributed deep in the body tissue in a networked pattern so that oxygen and a nutrient can be supplied to every corner of a periphery of the body. The capillary is composed of a single layer of vascular endothelial cells that forms a luminal structure, and a pericyte (vascular pericyte) surrounding the same. The pericyte, as a cell that covers a vascular endothelial cell, plays an important role in normal blood flow regulation such as maturing and stabilizing a blood vessel and maintaining the blood-brain barrier. There are critical lower limb ischemia and intractable ulcer as a serious disease that impairs blood flow, no effective drug therapy for these has been established, and the disease is treated by bypass surgery, endovascular treatment, or the like. In recent years, there has been a demand for the development of a new treatment method that induces the neogenesis of a peripheral capillary by a cell therapy (PTL 1).

"Basic fibroblast growth factor (bFGF)" is a member of the growth factor family, also referred to as FGF-2, is a protein having a variety of actions, and is known to contribute to angiogenesis by acting directly on vascular endothelial cells to promote vascular endothelial cell proliferation and lumen formation. Human bFGF/FGF-2 protein is a single-chain polypeptide having a molecular weight of 18 kD consisting of 154 amino acids, and is known to be released from a macrophage, an endothelial cell, a damaged muscle fiber, or the like (Henke C et al., Am. J. Pathol., (1993), 143: 1189-1199; Wang YX et al., J. Cell Sci., (2014), 127: 4543-4548). The most characteristic action of bFGF/FGF-2 in angiogenesis is to act directly on vascular endothelial cells to promote vascular endothelial cell proliferation and lumen formation. In addition, it is considered that bFGF/FGF-2 indirectly promotes angiogenesis by controlling the expression of a vascular endothelial growth factor (VEGF) in vascular smooth muscle cells (NPL 1). It has been reported that introduction of the bFGF/FGF-2 gene into ischemic muscle tissue using a Sendai virus vector enhances the endogenous VEGF and hepatocyte growth factor (HGF) expression, and induces improvement in lower limb ischemia (NPL 2). As described above, bFGF/FGF-2 is a potent angiogenic factor, and is expected to be applied to an ischemic disease.

### CITATION LIST

### PATENT LITERATURE

PTL 1: WO2008/104064

### NON PATENT LITERATURE

NPL 1: Marco Presta et al., Cytokine & Growth Factor Reviews,(2005), 16: 159-178
NPL 2: Ichiro Masaki et al., Circulation Research, (2002), 90: 966-973

### SUMMARY OF INVENTION

### TECHNICAL PROBLEM

An object of the present invention is to provide a cell therapy that is expected to be useful as an angiogenic therapy for a peripheral vascular disease such as critical lower limb ischemia. Specifically, the object is to provide a pericyte having high angiogenic potential and a method for producing the same.

### SOLUTION TO PROBLEM

In order to solve the above problem, the present inventors have carried out intensive studies such as introduction of several genes encoding an angiogenic factor into a pericyte and found that by introduction of a bFGF gene into a pericyte, the angiogenic potential of the pericyte transplanted into the living body is remarkably increased, and further, the pericyte is applicable to an angiogenic therapy for critical lower limb ischemia, or the like. The present invention has been completed based on such findings.

That is, the present invention has the following features:
[1] A pericyte introduced a basic fibroblast growth factor (bFGF) gene.
[2] The pericyte according to [1], wherein the pericyte is a primary pericyte.
[3] The pericyte according to [1], wherein the pericyte is a pericyte-like cell obtained by inducing differentiation of a pluripotent stem cell.
[4] The pericyte according to [3], wherein the pluripotent stem cell is a human pluripotent stem cell.
[5] The pericyte according to [3] or [4], wherein the pluripotent stem cell is an embryonic stem cell (ES cell) or an induced pluripotent stem cell (iPS cell).
[6] A pharmaceutical composition for an angiogenic therapy, including the pericyte according to any one of [1] to [5].
[7] The pharmaceutical composition according to [6], wherein the angiogenic therapy is a treatment for critical lower limb ischemia.
[8] The pharmaceutical composition according to [6] or [7], wherein the pharmaceutical composition is used in combination with a vascular endothelial cell.
[9] A pharmaceutical composition for an angiogenic therapy, including a combination of the pericyte according to any one of [1] to [5] and a vascular endothelial cell.
[10] The pharmaceutical composition according to [9], wherein the angiogenic therapy is a treatment for critical lower limb ischemia.
[11] A method for producing the pericyte according to any one of [1] to [5].
[12] An angiogenic therapy including administering a therapeutically effective amount of the pericyte according to any one of [1] to [5] to a subject.
[13] The angiogenic therapy according to [12], wherein the angiogenic therapy further includes administering a vascular endothelial cell.
[14] The angiogenic therapy according to [12] or [13], wherein the angiogenic therapy is a treatment for critical lower limb ischemia.
[15] Use of the pericyte according to any one of [1] to [5] in production of a pharmaceutical composition for an angiogenic therapy.
[16] Use of the pericyte according to any one of [1] to [5] in production of a pharmaceutical composition for treatment for critical lower limb ischemia.
[17] The pericyte according to any one of [1] to [5] for use in an angiogenic therapy.
[18] The pericyte according to any one of [1] to [5] for use in treatment for critical lower limb ischemia.
[19] A pericyte having enhanced expression of endogenous bFGF.

### ADVANTAGEOUS EFFECTS OF INVENTION

In the present invention, by introducing a bFGF gene into a pericyte, a pericyte having high angiogenic potential can be obtained and produced. In addition, a bFGF gene-introduced pericyte obtained by the method of the present invention can be used for an angiogenic therapy for critical lower limb ischemia or the like.

### BRIEF DESCRIPTION OF DRAWINGS

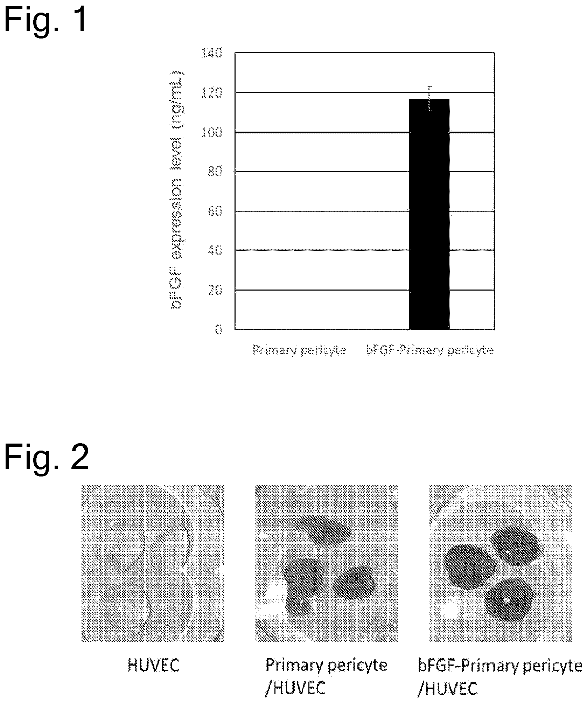

[Fig. 1] Fig. 1 shows results of evaluating the bFGF expression level in the bFGF gene-introduced human primary pericyte (bFGF-Primary pericyte) obtained in Example 3 together with the bFGF expression level in the human primary pericyte (Primary pericyte) in Example 4. The vertical axis represents the bFGF expression level (ng/mL). An error bar represents ± standard error of the mean.
[Fig. 2] Fig. 2 shows results of qualitatively evaluating the angiogenic potential of the bFGF gene-introduced human primary pericyte obtained in Example 3, in Example 5. Specifically, Fig. 2 shows results of administering a human umbilical vein endothelial cell (HUVEC), HUVEC and a human primary pericyte (Primary pericyte/HUVEC), or HUVEC and a bFGF gene-introduced human primary pericyte (bFGF-Primary pericyte/HUVEC) to an NOG mouse together with Matrigel, and recovering Matrigel from the mouse 14 days later and photographing the same.

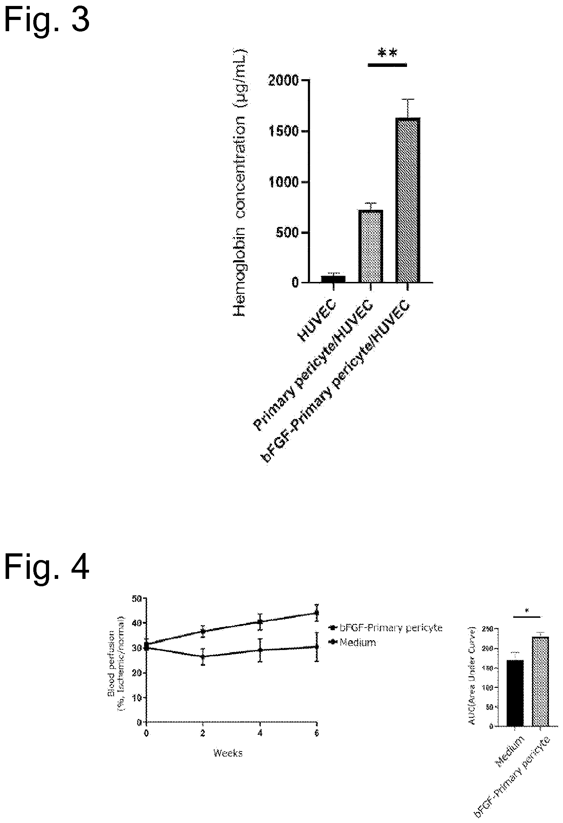

[Fig. 3] Fig. 3 shows results of quantitatively evaluating the angiogenic potential of the bFGF gene-introduced human primary pericyte obtained in Example 3, in Example 6. Specifically, Fig. 3 shows results of crushing each Matrigel recovered by the procedure of Example 5 and measuring the hemoglobin concentration of the supernatant after centrifugation. The vertical axis represents the hemoglobin concentration (µg/mL) of the Matrigel-derived supernatant. The P value represented by ** in Fig. 3 is 0.0001. An error bar represents ± standard error of the mean.
[Fig. 4] Fig. 4 shows a therapeutic effect of administering the bFGF gene-introduced human primary pericyte obtained in Example 3 to a lower limb ischemia model mouse, in Example 7. The horizontal axis in the left diagram of Fig. 4 represents the number of weeks after administration of the control medium (Medium) or the bFGF gene-introduced primary pericyte (bFGF-Primary pericyte) to an ischemic limb, and the vertical axis represents the blood flow ratio (%, Ischemic/normal) obtained by dividing a signal value of blood flow (Blood perfusion) in the ischemic limb by a signal value of blood flow in a normal limb. The vertical axis in the right diagram of Fig. 4 represents the AUC (Area Under Curve) after administration of the control medium or the bFGF gene-introduced primary pericyte, calculated based on the left diagram. The P value represented by ** in the right diagram of Fig. 4 is 0.0139. An error bar represents ± standard error of the mean.

### DESCRIPTION OF EMBODIMENTS

The present invention will be described in detail below.

### <Pericyte introduced bFGF gene>

The present invention provides a pericyte introduced a bFGF gene (also referred to as the "pericyte of the present invention").

The pericyte of the present invention is a pericyte introduced a bFGF gene. The nucleotide sequence of the bFGF gene and the amino acid sequence of bFGF have already been known, and the sequences have been published in a public database and the like. For example, the nucleotide sequence and the amino acid sequence of human bFGF have been published as GenBank Accession Number: M27968.1 and AAA52448.1, respectively. Specifically, human bFGF is encoded by the gene having GenBank Accession Number: M27968.1 (SEQ ID NO: 1) and has the amino acid sequence shown in GenBank Accession Number: AAA52448.1 (SEQ ID NO: 2). In the present invention, the bFGF gene introduced into a pericyte includes a gene encoding naturally occurring bFGF and also a gene encoding a variant having the function of bFGF. In one embodiment, the bFGF gene introduced into a pericyte in the present invention is a gene encoding a protein that has at least 80% or more, preferably 85% or more, 90% or more, 95% or more, or 98% or more identity to the amino acid sequence published as GenBank Accession Number: AAA52448.1 and that has the function as bFGF. In one embodiment, the bFGF gene introduced into a pericyte in the present invention is a gene encoding a protein that consists of an amino acid sequence derived from the amino acid sequence of SEQ ID NO: 2 by deletion, substitution, insertion, and/or addition of 1 to 5 amino acids, 1 to 4 amino acids, 1 to 3 amino acids, 1 to 2 amino acids, or 1 amino acid and that has the function as bFGF. In one embodiment, the bFGF gene introduced into a pericyte in the present invention is a gene encoding a protein that consists of the amino acid sequence shown in SEQ ID NO: 2. Having the function as bFGF can be confirmed by a known method (Beenken A & Mohammadi M, Nat. Rev. Drug Discov., (2009), 8: 235-253). In one embodiment, the bFGF gene introduced into a pericyte in the present invention may be a gene encoding bFGF having a secretion signal added thereto or a variant thereof. As the secretion signal, a secretion signal known to those skilled in the art can be used, and in one embodiment, the Bmp2/4 secretion signal (U.S. Patent US7816140) can be used. In one embodiment, the bFGF gene introduced into a pericyte in the present invention is a gene encoding a protein that has at least 80% or more, preferably 85% or more, 90% or more, 95% or more, or 98% or more identity to the amino acid sequence shown in SEQ ID NO: 4 and that has the function as bFGF. In one embodiment, the bFGF gene introduced into a pericyte in the present invention is a gene encoding a protein that consists of an amino acid sequence derived from the amino acid sequence of SEQ ID NO: 4. Herein, "bFGF" may also be referred to as "FGF-2."

For a nucleic acid sequence or an amino acid sequence, the term "identity" as used herein means a value Identity obtained by using parameters provided by default in a NEEDLE program (Needleman SB et al., J. Mol. Biol., (1970), 48: 443-453) search. The parameters are as follows.
Gap penalty = 10
Extend penalty = 0.5
Matrix = EBLOSUM62

### <<Pericyte>>

The term "pericyte" refers to a cell present in the brain, the periphery, the retina, or the like in such a way as to surround a microvascular wall or a capillary wall, and is also referred to as a vascular pericyte. The function thereof is as described above, and the pericyte, as a cell that covers a vascular endothelial cell, plays an important role in normal blood flow regulation such as maturating and stabilizing a blood vessel and maintaining the blood-brain barrier (Daneman R et al., Nature, (2010), 468: 562-568; Armulik A et al., Dev. Cell, (2011), 21: 193-215). When a cell function of a pericyte is impaired, an original function of the pericyte (for example, stabilization of a blood vessel, maintenance of blood flow, maintenance of the blood-brain barrier, or maintenance of the blood-nerve barrier) is impaired, leading to a serious blood flow disorder-related disease such as diabetic retinopathy. In addition, among skeletal muscle-derived pericytes, the presence of a cell referred to as a mesoangioblast, which has the ability to differentiate into a muscle or a bone, has been clarified (Gerli MFM et al., J. Vis. Exp., (2014), 83: 50523, Gerli MFM et al., Stem Cell Rep., (2019), 12: 461-473, Shimatani K et al., Am. J. Physiol. Heart Circ. Physiol., (2021), on line: https://doi.org/10.1152/ajpheart.00470.2020).

### -Primary pericyte-

In one embodiment, the pericyte of the present invention is a primary pericyte introduced a bFGF gene.

As used herein, the term "primary pericyte" means a pericyte directly collected from an individual organism, or a primary cultured cell or a subcultured cell obtained by culturing and proliferating the pericyte in vitro. A method for isolating a primary pericyte from an individual organism and a method for culturing the same are disclosed in, for example, Quattrocelli M et al., Methods Mol. Biol., (2012), 798: 65-76. The primary pericyte in the present invention is not particularly limited, and in one embodiment, the primary pericyte is a human primary pericyte. As the human primary pericyte, for example, it is desirable to use a human primary pericyte from a patient himself/herself who is a human, or a primary pericyte having the same or substantially the same human leukocyte antigen (HLA) genotype as the genotype of an individual who is a transplant recipient, from the viewpoint of avoiding rejection. Here, the term "substantially the same" means that the HLA genotypes match each other to the extent that an immune reaction to the transplanted pericyte can be suppressed by an immunosuppressive agent, and for example, such a pericyte is a pericyte having an HLA type in which 3 loci of HLA-A, HLA-B, and HLA-DR or 4 loci consisting of these 3 loci and HLA-C are matched.

### -Pericyte-like cell-

In one embodiment, the pericyte of the present invention is a pericyte-like cell obtained by inducing differentiation of a pluripotent stem cell introduced a bFGF gene.

As used herein, the term "pericyte-like cell" means a cell that is obtained by inducing differentiation of a pluripotent stem cell and has properties similar to those of a primary pericyte. It can be confirmed by a known method that a pericyte-like cell has properties similar to those of a pericyte (Armulik A et al., Dev. Cell, (2011), 21: 193-215). The induction of differentiation of a pluripotent stem cell into a pericyte-like cell can be carried out by using a method known to those skilled in the art (WO2013/108039, WO2009/156151, US9771561, US9868939, US2015/0368609, US2017/0342384, US2019/0316094). For example, the induction of differentiation of a pluripotent stem cell into a pericyte-like cell can be carried out by using the method described in the section <<Method for inducing differentiation of pluripotent stem cell into pericyte-like cell>> described later.

### «Pluripotent stem cell»

As used herein, the term "pluripotent stem cell" means a stem cell that has pluripotency by which the cell can differentiate into many cells having different properties and morphologies that are present in the living body, and that also has proliferative potential. A preferred pluripotent stem cell in the present invention is a human pluripotent stem cell. In one embodiment, the pericyte of the present invention is a pericyte-like cell obtained by inducing differentiation of a human pluripotent stem cell introduced a bFGF gene.

The pluripotent stem cell used in the present invention is not particularly limited, and examples thereof include an embryonic stem cell (ES cell), an embryonic stem cell prepared by using a nuclear transfer technique (nuclear transfer embryonic stem cell; ntES cell), a sperm stem cell (germline stem cell; GS cell), an embryonic germ cell (EG cell), an induced pluripotent stem cell (iPS cell), and a pluripotent cell derived from a cultured fibroblast or bone marrow stem cell (multi-lineage differentiating stress enduring cell; Muse cell). A preferred pluripotent stem cell for inducing differentiation into a pericyte-like cell in the present invention is an ES cell or an iPS cell. In one embodiment, the pericyte of the present invention is a pericyte-like cell obtained by inducing differentiation of an embryonic stem cell (ES cell) or an induced pluripotent stem cell (iPS cell) introduced a bFGF gene. In one embodiment, the pericyte of the present invention is a pericyte-like cell obtained by inducing differentiation of a human ES cell or a human iPS cell introduced a bFGF gene.

### -ES cell-

An ES cell is a stem cell that is established from the inner cell mass of an early embryo (for example, a blastocyst) of a mammal such as a human or a mouse and that has pluripotency and proliferative potential through self-renewal. The ES cell can be established by taking out the inner cell mass from the blastocyst of a fertilized egg of a target animal and culturing the inner cell mass on a fibroblast feeder. In addition, the maintenance of a cell by subculture can be carried out by using a medium supplemented with a substance such as leukemia inhibitory factor (LIF) or bFGF. A human ES cell can be established and maintained by a known method (disclosed in, for example, Suemori H et al., Biochem. Biophys. Res. Commun., (2006), 345: 926-932, or Kawasaki H et al., Proc. Natl. Acad. Sci. USA, (2002), 99: 1580-1585).

### -iPS cell-

An iPS cell is a general term for a pluripotent stem cell line that is artificially induced by introducing a specific gene into a somatic cell having lost the pluripotency thereof. A method for producing an iPS cell is known in the art and can be produced by introducing a reprogramming factor into an arbitrary somatic cell. Here, examples of the reprogramming factor include a gene product such as Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, or Glis1, and these reprogramming factors may be used singly, or a combination thereof may be used. Examples of the combination of the reprogramming factors include the combinations disclosed in WO2007/069666; WO2008/118820; WO2009/007852; WO2009/032194; WO2009/058413; WO2009/057831; WO2009/075119; WO2009/079007; WO2009/091659; WO2009/101084; WO2009/101407; WO2009/102983; WO2009/114949; WO2009/117439; WO2009/126250; WO2009/126251; WO2009/126655; WO2009/157593; WO2010/009015; WO2010/033906; WO2010/033920; WO2010/042800; WO2010/050626; WO2010/056831; WO2010/068955; WO2010/098419; WO2010/102267; WO2010/111409; WO2010/111422; WO2010/115050; WO2010/124290; WO2010/147395; WO2010/147612; Huangfu D et al., Nat. Biotechnol., (2008), 26: 795-797; Shi Y et al., Cell Stem Cell, (2008), 2: 525-528; Eminli S et al., Stem Cells, (2008), 26: 2467-2474; Huangfu D et al., Nat. Biotechnol., (2008), 26: 1269-1275; Shi Y et al., Cell Stem Cell, (2008), 3: 568-574; Zhao Y et al., Cell Stem Cell, (2008), 3: 475-479; Marson A Cell Stem Cell, (2008), 3: 132-135; Feng B et al., Nat. Cell Biol., (2009), 11: 197-203; Judson RL et al., Nat. Biotechnol., (2009), 27: 459-461; Lyssiotis CA et al., Proc. Natl. Acad. Sci. USA, (2009), 106: 8912-8917; Kim JB et al., Nature, (2009), 461: 649-643; Ichida JK et al., Cell Stem Cell, (2009), 5: 491-503; Heng JC et al., Cell Stem Cell, (2010), 6: 167-174; Han J et al., Nature, (2010), 463: 1096-1100; Mali P et al., Stem Cells, (2010), 28: 713-720; Maekawa M et al., Nature, (2011), 474: 225-229.

The somatic cell used for the production of an iPS cell may be either of a somatic cell of a neonate and a somatic cell of a healthy human or a patient, and is not particularly limited to these. In addition, any of a primary cultured cell, a passaged cell, and an established cell derived therefrom may be used. In an embodiment, examples of the somatic cell used for the production of an iPS cell include (1) a tissue stem cell (somatic stem cell) such as a neural stem cell, a hematopoietic stem cell, a mesenchymal stem cell, or a dental pulp stem cell, (2) a tissue progenitor cell, and (3) a differentiated cell present in an organ or a tissue such as a blood cell (peripheral blood cell, umbilical cord blood cell, or the like), a muscle cell, a skin cell, a hair cell, a liver cell, a gastric mucosa cell, an enterocyte, a splenic cell, a pancreatic cell, a brain cell, a lung cell, a kidney cell, and an adipocyte.

When an iPS cell is used as a material for inducing differentiation into a pericyte-like cell, it is desirable to use an iPS cell derived from a somatic cell of an individual who is a transplant recipient having the same or substantially the same human leukocyte antigen (HLA) genotype from the viewpoint of avoiding rejection, but this is not the only option. Here, the term "substantially the same" means that the HLA genotypes match each other to the extent that an immune reaction to the transplanted cell can be suppressed by an immunosuppressive agent, and for example, such a cell is an iPS cell derived from a somatic cell having an HLA type in which 3 loci of HLA-A, HLA-B, and HLA-DR or 4 loci consisting of these 3 loci and HLA-C are matched.

As a pluripotent stem cell, which is a material for inducing a pericyte-like cell, a pluripotent stem cell that does not cause a rejection reaction in allotransplantation prepared by the method disclosed in, for example, Gornalusse GG et al., Nat. Biotechnol., (2017), 35: 765-772 can also be used. The pluripotent stem cell that do not cause a rejection reaction in allotransplantation is preferably an ES cell or an iPS cell that does not cause a rejection reaction in allotransplantation, and more preferably a human ES cell or a human iPS cell that does not cause a rejection reaction in allotransplantation. In one embodiment, the pericyte of the present invention is a pericyte-like cell obtained by inducing differentiation of a pluripotent stem cell that does not cause a rejection reaction in allotransplantation introduced a bFGF gene. In one embodiment, the pericyte of the present invention is a pericyte-like cell obtained by inducing differentiation of a human ES cell or a human iPS cell that does not cause a rejection reaction in allotransplantation introduced a bFGF gene.

### <Pericyte having enhanced expression of endogenous bFGF>

In one embodiment, the pericyte of the present invention is a pericyte having enhanced endogenous bFGF expression. Here, the term "enhanced endogenous bFGF expression" encompasses both of direct enhancement of endogenous bFGF activity due to increased expression level of the endogenous bFGF protein and indirect enhancement of endogenous bFGF activity due to release of a related inhibitory system, or the like, in a pericyte-like cell obtained by inducing differentiation of a primary pericyte or a pluripotent stem cell. The enhancement of endogenous bFGF expression can be brought about by induction of endogenous bFGF expression by an external factor. Examples of a method for inducing endogenous bFGF expression include a method for induction by acidosis (D'Arcangelo D et al., Circ. Res., (2000), 86: 312-318). The expression level of the bFGF protein in a pericyte having enhanced endogenous bFGF expression is not particularly limited. In a normal primary pericyte, the bFGF expression was below the detection limit (see Example 4).

### <Method for producing pericyte introduced bFGF gene>

In addition, the present invention provides a method for producing a pericyte introduced a bFGF gene (also referred to as the production method of the present invention").

In one embodiment, the production method of the present invention includes introducing a bFGF gene into a pericyte. In one embodiment, the production method of the present invention includes introducing a bFGF gene into a primary pericyte. In one embodiment, the production method of the present invention includes introducing a bFGF gene into a pericyte-like cell obtained by inducing differentiation of a pluripotent stem cell. In one embodiment, the production method of the present invention includes introducing a bFGF gene into a pericyte-like cell obtained by inducing differentiation of an ES cell or an iPS cell. In one embodiment, the production method of the present invention includes introducing a bFGF gene into a pericyte-like cell obtained by inducing differentiation of a human pluripotent stem cell. In one embodiment, the production method of the present invention includes introducing a bFGF gene into a pericyte-like cell obtained by inducing differentiation of a human ES cell or a human iPS cell.

In one embodiment, the production method of the present invention includes introducing a bFGF gene into a pluripotent stem cell, and inducing differentiation of the pluripotent stem cell introduced the bFGF gene into a pericyte-like cell. In one embodiment, the production method of the present invention includes introducing a bFGF gene into an ES cell or an iPS cell, and inducing differentiation of the ES cell or the iPS cell introduced the bFGF gene into a pericyte-like cell. In one embodiment, the production method of the present invention includes introducing a bFGF gene into a human pluripotent stem cell, and inducing differentiation of the human pluripotent stem cell introduced the bFGF gene into a pericyte-like cell. In one embodiment, the production method of the present invention includes introducing a bFGF gene into a human ES cell or a human iPS cell, and inducing differentiation of the human ES cell or the human iPS cell introduced the bFGF gene into a pericyte-like cell.

### [Method for introducing bFGF gene into cell]

A bFGF gene can be prepared by using a method known in the art based on nucleotide sequence information. For example, the bFGF gene can be synthesized by using a gene synthesis method known in the art.

In the present invention, as the method for introducing a bFGF gene into a pericyte or a pluripotent stem cell, a method commonly used for transfection of an animal cell, such as a calcium phosphate method, a lipofection method, an electroporation method, a microinjection method, or a method for introduction using a virus vector can be used. In one embodiment, as the method for introducing a bFGF gene into a pericyte or a pluripotent stem cell, a method for introduction using a virus vector can be used. Examples of the virus vector that can be used to introduce a bFGF gene into a pericyte or a pluripotent stem cell include a lentivirus, an adenovirus, an adeno-associated virus, or a retrovirus. In one embodiment, as the method for introducing a bFGF gene into a pericyte or a pluripotent stem cell, a method for introduction using a lentivirus vector can be used. Specifically, as described in Example 3, by infecting a cell with a lentivirus for bFGF gene introduction, a bFGF gene can be introduced into the cell.

### [Method for inducing differentiation of pluripotent stem cell into pericyte-like cell]

In the present invention, a pericyte-like cell can be obtained by inducing differentiation of a pluripotent stem cell into a pericyte-like cell, as described above. The method for inducing differentiation of a pluripotent stem cell into a pericyte-like cell is not particularly limited, and in one embodiment, a pericyte-like cell can be obtained by a method including the following steps (a) and (b):
(a) a step of differentiating a pluripotent stem cell into an early mesodermal cell; and
(b) a step of differentiating the early mesodermal cell obtained in step (a) into a pericyte-like cell.

### Steps (a) and (b) will be described below.

Step (a) is a step of differentiating a pluripotent stem cell into an early mesodermal cell (primitive posterior mesoderm). In the present invention, the method for differentiating a pluripotent stem cell into an early mesodermal cell is not particularly limited, and the method for inducing differentiation of a pluripotent stem cell into an early mesodermal cell disclosed in, for example, US9,868,939, US9,771,561, or Uenishi G et al., Stem Cell Reports, (2014), 3: 1073-1084 can be used. The differentiation of a pluripotent stem cell into an early mesodermal cell can be confirmed by using a surface antigen marker (PDGFRα, APLNR, or the like) specific to an early mesodermal cell as disclosed in, for example, Vodyanik MA et al., Cell Stem Cell, (2010), 7: 718-729, US9,771,561, and Uenishi G et al., Stem Cell Reports, (2014), 3: 1073-1084.

Step (b) is a step of inducing differentiation of the early mesodermal cell obtained in the step (a) into a pericyte-like cell. In the present invention, the method for differentiating the early mesodermal cell into a pericyte-like cell is not particularly limited, and the method for inducing differentiation of an early mesodermal cell into a pericyte-like cell disclosed in, for example, US9,868,939 can be used. The differentiation of the early mesodermal cell into a pericyte-like cell can be confirmed by using a surface antigen marker such as NG2 or CD146 (Herrmann M. et al., Eur. Cells Mater., (2016), 31: 236-249, Covas DT. et al., Exp. Hematol., (2008), 36: 642-654, Lv FJ. et al., Stem Cells, (2014), 32: 1408-1419).

In one embodiment, in step (b), a spheroid of the early mesodermal cell is first formed, and then it is possible to induce differentiation of the spheroid of the early mesodermal cell into a pericyte-like cell.

In the present invention, the method for forming a spheroid of the early mesodermal cell is not particularly limited, and a known method can be used. Examples thereof include the method using a methylcellulose medium disclosed in US9,771,561. The composition of a spheroid formation medium can be set with reference to a known technique (Vodyanik MA et al., Cell Stem Cell, (2010), 7: 718-729, or the like).

In the present invention, the method for differentiating the early mesodermal cell that has formed a spheroid into a pericyte-like cell is not particularly limited, and a known method can be used. For example, the method disclosed in US9,868,939 can be used.

### [Method for culturing bFGF gene-introduced pericyte]

The method for culturing and proliferating the pericyte of the present invention is not particularly limited, and a method for culturing and proliferating a pericyte known in the art can be used.

The medium for culturing the pericyte of the present invention is not particularly limited as long as it is a medium suitable for culturing a pericyte, and MEM medium, BME medium, D-MEM medium, α-MEM medium, IMEM medium, ES medium, DM-160 medium, Fisher medium, F12 medium, WE medium, RPMI medium, StemSpan medium, StemPro medium, and a mixture thereof can be used as a basal medium.

The medium for culturing the pericyte of the present invention may be appropriately supplemented with various nutrient sources necessary for cell maintenance and proliferation. The medium can include, for example as a nutrient source, a carbon source such as glycerol, glucose, fructose, sucrose, lactose, honey, starch, or dextrin; a hydrocarbon such as fatty acid, oil/fat, lecithin, or an alcohol; a nitrogen source such as ammonium sulfate, ammonium nitrate, ammonium chloride, urea, or sodium nitrate; an inorganic salt such as common salt, a potassium salt, a phosphate, a magnesium salt, a calcium salt, an iron salt, or a manganese salt; monopotassium phosphate, dipotassium phosphate, magnesium sulfate, sodium chloride, ferrous sulfate, sodium molybdate, sodium tungstate, and manganese sulfate; various vitamins; an amino acid; or the like. In one embodiment, an amino acid such as glutamine can be used as a nutrient source added to the medium for culturing the pericyte of the present invention.

The medium for culturing the pericyte of the present invention may be appropriately supplemented with a growth factor such as an FGF family growth factor necessary for cell proliferation. The growth factor to be added is not particularly limited, and in one embodiment, bFGF or modified bFGF having enhanced thermostability can be used as a growth factor to be added to the medium for culturing the pericyte of the present invention.

The pH of the medium for culturing the pericyte of the present invention is in the range of 5.5 to 9.0, preferably 6.0 to 8.0, and more preferably 6.5 to 7.5.

The pericyte is an adherent cell that has the property of adhering to an extracellular matrix to proliferate. Because of this, in one embodiment, a suitable scaffold can be used in culturing the pericyte of the present invention. The scaffold is not particularly limited as long as it is a matrix, a substrate, or a carrier to which a cell can adhere to divide and proliferate, and examples thereof include fibronectin, vitronectin, collagen, proteoglycan, laminin, tenascin, entactin, elastin, fibrillin, hyaluronic acid, gelatin, poly-L-lysine, and poly-D-lysine. In one embodiment, a collagen matrix can be used as a scaffold for use in culturing the pericyte of the present invention.

In one embodiment, the culture of the pericyte of the present invention can be carried out at 36°C to 38°C. In one embodiment, the culture of the pericyte of the present invention can be carried out at 36.5°C to 37.5°C in an atmosphere of 1% to 25% O₂ and 1% to 15% CO₂ while appropriately exchanging the medium.

### <Pharmaceutical composition and the like of the present invention>

The present invention also provides a pharmaceutical composition including the pericyte of the present invention (also referred to as the "pharmaceutical composition of the present invention"). The pharmaceutical composition can be prepared by a commonly used method by using an excipient commonly used in the art, that is, a pharmaceutical excipient, a pharmaceutical carrier, or the like. In formulating the pharmaceutical composition, an excipient, a carrier, an additive, or the like according to the dosage form therefor can be used in a pharmaceutically acceptable range. In one embodiment, the pharmaceutical composition of the present invention includes a primary pericyte introduced a bFGF gene. In one embodiment, the pharmaceutical composition of the present invention includes a pericyte-like cell obtained by inducing differentiation of a pluripotent stem cell introduced a bFGF gene. The pharmaceutical composition of the present invention includes a pericyte-like cell obtained by inducing differentiation of an ES cell or an iPS cell introduced a bFGF gene. In one embodiment, the pharmaceutical composition of the present invention includes a pericyte-like cell obtained by inducing differentiation of a human pluripotent stem cell introduced a bFGF gene. In one embodiment, the pharmaceutical composition of the present invention includes a pericyte-like cell obtained by inducing differentiation of a human ES cell or a human iPS cell introduced a bFGF gene.

In one embodiment, the pharmaceutical composition of the present invention is a pharmaceutical composition for an angiogenic therapy. The pharmaceutical composition encompasses a therapeutic agent for an angiogenic therapy including the pericyte of the present invention. The angiogenic therapy is a treatment method that, in order to increase the amount of oxygen-rich blood reaching an organ, a tissue, or a site of the body that is in an ischemic state, promotes the formation of a new blood vessel in the organ, tissue, or site of the body that is in an ischemic state. The angiogenic therapy includes a treatment for a peripheral vascular disease such as critical lower limb ischemia or diabetic retinopathy, or a disease such as pulmonary hypertension or intractable ulcer. In one embodiment, the pharmaceutical composition of the present invention is a pharmaceutical composition for treatment for a peripheral vascular disease such as critical lower limb ischemia or diabetic retinopathy, pulmonary hypertension, intractable ulcer, or the like. In one embodiment, the pharmaceutical composition of the present invention is a pharmaceutical composition for treatment for critical lower limb ischemia.

The present invention provides use of the pericyte of the present invention in production of a pharmaceutical composition for an angiogenic therapy. The present invention provides the pericyte of the present invention for use in an angiogenic therapy. In addition, the present invention provides use of the pericyte of the present invention for an angiogenic therapy.

The present invention provides use of the pericyte of the present invention in production of a pharmaceutical composition for treating critical lower limb ischemia. The present invention provides the pericyte of the present invention for use in treatment for critical lower limb ischemia. In addition, the present invention provides use of the pericyte of the present invention for treatment for critical lower limb ischemia.

The present invention also provides an angiogenic therapy including administering a therapeutically effective amount of the pericyte of the present invention to a subject (also referred to as "the treatment method of the present invention"). In the treatment method of the present invention, the "subject" is a human or a non-human animal in need of treatment. In one embodiment, the "subject" is a human in need of the treatment method. When the pericyte of the present invention is administered to a human, it can be administered to the subject in the form of a pharmaceutical composition including the pericyte of the present invention and a pharmaceutically acceptable excipient. The dosage and frequency of administration of the pharmaceutical composition of the present invention to a human can be appropriately regulated according to the disease to be treated, the severity thereof, the age, body weight, and condition of the human to be treated, and the like.

The method for administering the pharmaceutical composition of the present invention is not particularly limited, and depending on the site of application, local transplantation by surgical means, intravenous administration, lower limb puncture administration, local infusion administration, subcutaneous administration, intradermal administration, intramuscular administration, or the like can be considered.

The pharmaceutical composition of the present invention may be formed into a sheet and applied directly to an affected area. The sheet may include not only a cell but also a suitable support.

The pharmaceutical composition of the present invention may include a scaffold material or a component that assists in cell maintenance or proliferation, administration to an affected area, and a different pharmaceutically acceptable carrier. Examples of the component necessary for cell maintenance or proliferation include a medium component such as a carbon source, a nitrogen source, a vitamin, a mineral, a salt, or various cytokines, and an extracellular matrix preparation such as Matrigel.

The pericyte of the present invention or the pharmaceutical composition of the present invention can also be used in combination with a vascular endothelial cell. As used herein, the term "use in combination" means administering a plurality of active pharmaceutical ingredients simultaneously or separately to the same subject. For use in combination, the plurality of active pharmaceutical ingredients may be included in the same composition, or may be included separately in different compositions. In one embodiment, the pharmaceutical composition of the present invention is a pharmaceutical composition used in combination with a vascular endothelial cell. In one embodiment, the pharmaceutical composition of the present invention is a pharmaceutical composition further including a vascular endothelial cell. In one embodiment, the treatment method of the present invention further includes administering a vascular endothelial cell.

The present invention also includes a pharmaceutical composition including a combination of the pericyte of the present invention and a vascular endothelial cell. As used herein, the term "including a combination of" means that a plurality of active pharmaceutical ingredients are included in the same pharmaceutical composition, or a plurality of active pharmaceutical ingredients are included separately in different pharmaceutical compositions. In one embodiment, the pharmaceutical composition including a combination of the pericyte of the present invention and a vascular endothelial cell is a pharmaceutical composition including the pericyte of the present invention and a vascular endothelial cell. In one embodiment, a pharmaceutical composition including a combination of the pericyte of the present invention and a vascular endothelial cell is a combination of pharmaceutical compositions in which the pericyte of the present invention and a vascular endothelial cell are separately included in different pharmaceutical compositions. In one embodiment, the pharmaceutical composition including a combination of the pericyte of the present invention and a vascular endothelial cell is a combination of a pharmaceutical composition including the pericyte of the present invention and a pharmaceutical composition including a vascular endothelial cell. In one embodiment, the pharmaceutical composition including a combination of the pericyte of the present invention and a vascular endothelial cell is a pharmaceutical composition for an angiogenic therapy. In one embodiment, the pharmaceutical composition including a combination of the pericyte of the present invention and a vascular endothelial cell is a pharmaceutical composition for treating critical lower limb ischemia.

### «Vascular endothelial cell»

A "vascular endothelial cell" is one layer of a flat cell that lines the vascular lumen, and has various functions such as regulation of vascular tonicity and vascular permeability, angiogenesis, anti-inflammation, and promotion of blood coagulation. A large blood vessel at the level of an artery or a vein has a three-layer structure composed of the intima, the media, and the adventitia, each of which is mainly composed of a vascular endothelial cell, a smooth muscle cell, and a fibroblast. On the other hand, in a small blood vessel at the capillary level, the luminal structure of a vascular endothelial cell is surrounded by pericytes. In a mature capillary, a pericyte shares the basement membrane with a vascular endothelial cell and is present in the form of being embedded therein. In recent years, it has been known that a pericyte and a vascular endothelial cell carry out cell signaling mutually to regulate differentiation and proliferation and play an important role in the maturation, stabilization, and maintenance of a capillary, formation of the basement membrane, deposition of an extracellular matrix, and the like.

The vascular endothelial cell that can be combined with the pericyte of the present invention or the pharmaceutical composition of the present invention is not particularly limited, and in one embodiment, the vascular endothelial cell is a primary vascular endothelial cell or a vascular endothelial cell obtained by inducing differentiation of a pluripotent stem cell or the like. The vascular endothelial cell that can be used in combination with the pericyte of the present invention or the pharmaceutical composition of the present invention is not particularly limited, and in one embodiment, the vascular endothelial cell is a primary vascular endothelial cell, or a vascular endothelial cell obtained by inducing differentiation of a pluripotent stem cell or the like. In the angiogenic therapy of the present invention, when the pericyte of the present invention and a vascular endothelial cell are used in combination, the vascular endothelial cell can be administered simultaneously with the administration of the pericyte of the present invention, or before the administration or after the administration of the pericyte of the present invention.

The term "primary vascular endothelial cell" means a vascular endothelial cell directly collected from an individual organism, or a primary cultured cell or a passaged cell obtained by culturing and proliferating the vascular endothelial cell in vitro. The primary vascular endothelial cell is not particularly limited, and in an embodiment, the primary vascular endothelial cell is a human primary vascular endothelial cell. As the human vascular endothelial cell, for example, it is desirable to use a primary vascular endothelial cell from a patient himself/herself who is a human, or an individual who is a transplant recipient having the same or substantially the same human leukocyte antigen (HLA) genotype as that of the patient from the viewpoint of avoiding rejection. Here, the term "substantially the same" means that the HLA genotypes match each other to the extent that an immune reaction to the transplanted vascular endothelial cell can be suppressed by an immunosuppressive agent, and for example, such a vascular endothelial cell is a vascular endothelial cell having an HLA type in which 3 loci of HLA-A, HLA-B, and HLA-DR or 4 loci consisting of these 3 loci and HLA-C are matched. In one embodiment, the vascular endothelial cell that can be used in combination with or combined with the pericyte of the present invention or the pharmaceutical composition of the present invention is a human primary vascular endothelial cell.

The "vascular endothelial cell obtained by inducing differentiation of a pluripotent stem cell or the like" that can be combined with the pericyte of the present invention or the pharmaceutical composition of the present invention is not particularly limited, and a vascular endothelial cell produced by the method disclosed in, for example, Ikuno T et al., Pros One, (2019), 12: e0173271 or Cho SW et al., Circulation, (2007), 116: 2409-2419 can be used. In one embodiment, the vascular endothelial cell that can be used in combination with or combined with the pericyte of the present invention or the pharmaceutical composition of the present invention is a vascular endothelial cell obtained by inducing differentiation of a human pluripotent stem cell or the like.

Specific Examples referred to in order to obtain a further understanding of the invention will be provided here, but these are for an illustrative purpose and do not limit the invention.

### EXAMPLES

### Example 1: Establishment of human primary pericyte derived from skeletal muscle

A portion of the human quadriceps femoris muscle was soaked in PBS, and the muscle in the soaked state was finely cut along the fiber thereof by using a scalpel and tweezers. The cut muscle fiber was placed in a 50 mL centrifuge tube (Corning, 352070), 15 mL of a collagenase solution (see below) was added, and the centrifuge tube was allowed to stand for 1 hour in a hot bath at 37°C. A cell suspension other than the muscle fiber was recovered and designated as cell suspension 1. The collagenase solution was again added in two portions of 15 mL each, and for each addition of the collagenase solution, the cell suspension was recovered by the same procedure as in cell suspension 1 to obtain cell suspension 2 and cell suspension 3. Cell suspensions 1, 2, and 3 were each passed through a 100 µm cell strainer (Corning, 352360), and the resulting cell suspensions were centrifuged under conditions of 300 g, 4°C, and 5 minutes. The supernatant was removed, 20 mL of a pericyte establishment medium (see below) was added to each centrifuge tube, dispensed into 2 collagen-coated dishes (Corning, 356450), and cultured at 37°C in an atmosphere of 5% CO₂ and 5% O₂. Cells derived from cell suspensions 1, 2, and 3 are referred to below as cells under condition 1, 2, and 3, respectively. On days 3, 6, and 8 of culture, each culture supernatant was removed, and 10 mL of the pericyte establishment medium was added. For the cell under condition 1, the culture supernatant on the dish was removed on day 13 of culture, washing with 5 mL of PBS was carried out, then 1 mL of a cell dissociation reagent (TrypLE Express, ThermoFisher Scientific, 12604013) was added, and the resultant was allowed to stand on a 37°C plate for 5 minutes. 4 mL of the pericyte establishment medium was added to the dish, the resulting cell suspension was recovered and centrifuged under conditions of 300 g, room temperature, and 5 minutes. The supernatant was removed, then 5 mL of CELLBANKER 1 (Takara Bio, CB011) was added, and 1 mL portions were dispensed into cryotubes and stored in liquid nitrogen.

The culture supernatants on the dishes for the cells under conditions 2 and 3 were removed on day 10 of culture, each thereof was washed with 5 mL of PBS, then TrypLE Express was added in 1 mL portions, and the resultant was allowed to stand on a 37°C plate for 5 minutes. 4 mL of the pericyte establishment medium was added to each dish, the cell suspension was recovered, the cell suspensions under conditions 2 and 3 were mixed together in the same centrifuge tube, and centrifuged under conditions of 300 g, room temperature, and 5 minutes. The supernatant was removed, then 5 mL of CELLBANKER 1 was added, and 1 mL portions were dispensed into cryotubes and stored in liquid nitrogen. At a later date, 9 mL of a pericyte growth medium (see below) was added to a 50 mL centrifuge tube, 1 mL of a CELLBANKER 1 solution including the thawed cells under conditions 2 and 3 above was added, and the resultant was centrifuged under conditions of 300 g, 4°C, and 5 minutes. The supernatant was removed, 20 mL of the pericyte growth medium was added to the centrifuge tube, and the cell suspension was recovered. The recovered cell suspension was dispensed into 2 collagen-coated dishes and cultured at 37°C in an atmosphere of 5% CO₂ and 5% O₂. On day 3 of culture, the culture supernatant on each dish was removed, and 10 mL of the pericyte growth medium was added. The next day, the culture supernatant was removed, and washing with 5 mL of PBS was carried out, 2 mL of a cell dissociation reagent (Accutase (registered trademark), Innovative Cell Technologies, AT104) was added, and the resultant was allowed to stand on a 37°C plate for 5 minutes. 8 mL of the pericyte establishment medium was added to each dish, and the resulting cell suspension was recovered into a centrifuge tube and centrifuged under conditions of 300 g, room temperature, and 5 minutes. The supernatant was removed, 100 µL of PBS was added to the centrifuge tube, 20 µL of a blocking reagent (FcR Blocking Reagent, Miltenyi Biotec, 130-059-901) was added, and the centrifuge tube was allowed to stand on ice for 15 minutes. Subsequently, 20 µL each of an anti-human ALP (Alkaline Phosphatase) antibody (R&D Systems, FAB1448P) and an anti-CD56 APC antibody (Miltenyi Biotec, 130-090-843) were added, and the centrifuge tube was allowed to stand on ice for 20 minutes. Washing with PBS was carried out twice and then suspension in PBS was carried out again, and an ALP(+) and CD56(-) cells (that is, human primary pericyte) wer separated by using a cell sorter (SH800S, Sony). The separated cells were suspended in the pericyte growth medium, seeded on a collagen-coated dish, and cultured.

### [Collagenase solution]

The collagenase solution was prepared by dissolving 100 mg of Collagenase, Type II (ThermoFisher Scientific, 17101015) in 200 mL of TrypLE select (ThermoFisher Scientific, 12563029).

### [Pericyte establishment medium]

The composition is as follows.
- 92% MegaCell Dulbecco's Modified Eagle's Medium (Sigma-Aldrich, M3942)
- 5% FBS (Sigma-Aldrich)
- 1% GlutaMax (ThermoFisher Scientific, 35050061)
- 1% MEM Non-essential Amino Acid Solution (Sigma-Aldrich, M7145)
- 1% Penicillin-Streptomycin (Sigma-Aldrich, P0781)
- 100 µM 2-Mercaptoethanol (ThermoFisher Scientific, 21985023)
- 5ng/mL FGF-basic (154a.a.), Human, Recombinant (PeproTech, 100-18B)

### [Pericyte growth medium]

The composition is as follows.
- 77% MegaCell Dulbecco's Modified Eagle's Medium
- 20% FBS
- 1% GlutaMax
- 1% MEM Non-essential Amino Acid Solution
- 1% Penicillin-Streptomycin
- 100 µM 2-Mercaptoethanol
- 5ng/mL Animal-free Recombinant Human FGFbasic-TS (Proteintech, HZ-1285)

### Example 2: Preparation of lentivirus vector for bFGF gene introduction

5 × 10⁶ cells of a cell line for lentiviral packaging (Lenti-X 293T Cell Line, Takara Bio, 632180) were seeded by using 10 mL of a 293T growth medium (see below) per 10 cm dish, and the cells seeded in the 4 dishes were cultured at 37°C in an atmosphere of 5% CO₂. The next day, 28 µL of Lentiviral High Titer Packaging Mix (Takara Bio, 6194) and 15 µL of a bFGF-inserted plasmid (see below) were added to 6 mL of D-MEM (FUJIFILM Wako Pure Chemical Corporation, 045-30285), mixed, and allowed to stand at room temperature for 5 minutes. Further, 180 µL of a transfection reagent (TransIT-293 Transfection Reagent, Takara Bio, MIR2704) was added thereto, mixed, and allowed to stand at room temperature for 30 minutes (hereinafter referred to as the "Transfection solution"). 1.5 mL of the Transfection solution per 10 cm dish seeded with the Lenti-X 293T Cell Line the day before was added to each of the 4 dishes. The next day, the supernatant on each dish was removed and 10 mL of the 293T growth medium was added. After 2 days, the culture supernatant was recovered, and centrifuged under conditions of room temperature, 300 g, and 5 minutes in order to remove a detached cell. The supernatant was recovered, a lentivirus concentration reagent (Lenti-X Concentrator, Takara Bio, 631231) was added thereto at an amount of 1/3 of the amount of the supernatant, and the resultant was centrifuged under conditions of 500 g, 4°C, and 45 minutes. The supernatant was removed, and 200 µL of D-MEM was added to obtain a lentivirus suspension for bFGF gene introduction.

### [293T Growth medium]

The composition is as follows.
- D-MEM
- 10% FBS
- 1% GlutaMAX

### [bFGF-Inserted plasmid]

Prepared was a plasmid (pLe6-Bmp-bFGF: SEQ ID NO: 5) in which a gene sequence (SEQ ID NO: 3) encoding the amino acid sequence (SEQ ID NO: 4) of Bmp-bFGF (Patent Literature US 7816140 B2) was inserted by using pLenti6/V5 Directional TOPO Cloning Kit (ThermoFisher Scientific, K495510).

### Example 3: Preparation of bFGF gene-introduced human primary pericyte

The human primary pericyte established in Example 1 was seeded in 2 wells on a collagen-coated 6-well plate (Iwaki, 4810-010) at a cell count of 1 × 10⁵ cells/well in a pericyte growth medium. After 2 days, 1.2 µL of a 10 mg/mL polybrene solution (Nacalai Tesque, 12996-81) and 62 µL of the lentivirus suspension for bFGF gene introduction prepared in Example 2 were added to 3 mL of the pericyte growth medium, mixed, and allowed to stand at room temperature for 5 minutes (suspension A). The supernatant of the cultured human primary pericyte was removed, 1.5 mL of suspension A per well was aspirated and added to each of the 2 wells. The plate was centrifuged under conditions of room temperature, 1200 g, and 60 minutes and then the resultant was cultured at 37°C in an atmosphere of 5% CO₂ and 5% O₂. The next day, the supernatant was removed, washing with PBS was carried out nine times, then 1 mL of Accutase was added, and the resultant was allowed to stand on a 37°C plate for 5 minutes. 4 mL of the pericyte growth medium was added to a dish, the cell suspension was recovered, and the cell suspensions for 2 wells were recovered into the same centrifuge tube and centrifuged under conditions of 300 g, room temperature, and 5 minutes. The supernatant was removed, 10 mL of the pericyte growth medium was added, and the recovered cell was seeded on a collagen-coated dish and cultured. The culture was carried out at 37°C in an atmosphere of 5% CO₂ and 5% O₂. The next day, 10 mg/mL Blasticidin S Solution (FUJIFILM Wako Pure Chemical Corporation, 026-18711) was added to the collagen dish in such a way as to reach a final concentration of 2.5 µg/mL, and the culture was further continued. The cell proliferated through the present operation was used as a bFGF gene-introduced human primary pericyte.

### Example 4: Quantitation of bFGF expression level in bFGF gene-introduced human primary pericyte

The bFGF gene-introduced human primary pericyte prepared in Example 3 and the human primary pericyte (control) prepared in Example 1 were seeded on a collagen-coated dish at a cell count of 3 × 10⁵ cells/dish by using a pericyte growth medium, and cultured at 37°C in an atmosphere of 5% CO₂ and 5% O₂. On day 3 of culture, the culture supernatant was recovered and passed through a syringe filter (IWAKI, 2053-025). The concentration of bFGF in the supernatant passed through the syringe filter was measured by using an ELISA kit (Human FGF basic Quantikine ELISA KIT, R&D Systems, DFB50). The measurement of the bFGF concentration was carried out according to the protocol attached to the kit.

In the control human primary pericyte, no bFGF expression was able to be detected, whereas in the bFGF gene-introduced human primary pericyte, very high bFGF expression was observed (Fig. 1).

### Example 5: Qualitative evaluation of angiogenic potential of bFGF gene-introduced human primary pericyte

Human Umbilical Vein Endothelial Cells (HUVEC, PromoCell, C-12200) were cultured at 37°C in an atmosphere of 5% CO₂ by using an endothelial cell growth medium (PromoCell, C-22111). In addition, the human primary pericyte (control) established in Example 1 and the bFGF gene-introduced human primary pericyte prepared in Example 3 were each cultured by using a pericyte growth medium on a collagen-coated dish. After confirming that each of the three cells proliferated to a confluent state, the supernatant was removed, washing with PBS was carried out, then 2 mL of Accutase was added, and the resultant was allowed to stand on a 37°C plate for 5 minutes. Next, 8 mL of an endothelial cell growth medium for the HUVEC, and a pericyte growth medium for the human primary pericyte and the bFGF gene-introduced human primary pericyte was added to each dish. Cell suspension was collected from each dish, centrifuged at 300g at room temperature for 5 minutes, and the supernatant was removed. HUVEC was suspended in endothelial cell growth medium and the two types of human primary pericyte were suspended in pericyte growth medium. 5.5 × 10⁵ cells of the HUVEC were added to each of nine of 1.5 mL tubes (Eppendorf, 0030120.086), and further, to 3 tubes thereof the human primary pericyte was added and to another 3 tubes thereof the bFGF gene-introduced human primary pericyte was added, each at 5.5 × 10⁵ cells per tube, and admixed. Each tube was centrifuged under conditions of 300 g, 4°C, and 5 minutes to remove the supernatant, further, washing with PBS was carried out once, and then the tube was centrifuged again under the same conditions to remove the supernatant. Next, 400 µL of an extracellular matrix (Matrigel (registered trademark) Growth factor reduced, Corning, 356231, hereinafter referred to as "Matrigel") was added to each tube, admixed on ice, and then Matrigel including the cell was aspirated with a syringe equipped with a 25 gauge needle. To 79 mL of physiological saline (Otsuka Pharmaceutical Factory, Inc.), 3 mL of Domitor (Nippon Zenyaku Kogyo Co., Ltd.), 8 mL of Dormicum Injection (Maruishi Pharmaceutical Co., Ltd.), and 10 mL of Betorfal (Meiji Seika Pharma Co., Ltd.) were added to prepare a triple mixed anesthetic solution. 300 µL of the triple mixed anesthetic solution was administered to the abdominal cavity of each of 5 NOG mice (NOD.Cg-Prkdc^{scid}Il2rg^{tm1Sug}/ShiJic mice, In-Vivo Science). The whole amount of Matrigel including any of the HUVEC alone (HUVEC), the HUVEC and the control human primary pericyte (Primary pericyte/HUVEC), the HUVEC and the bFGF gene-introduced human primary pericyte (bFGF-Primary pericyte/HUVEC) prepared was subcutaneously administered to a total of 9 locations on the left side and the right side of the back of each of the 5 anesthetized mice. After 14 days, the administered Matrigel was recovered from each mouse and photographed (Fig. 2).

Matrigel including the bFGF gene-introduced human primary pericyte (bFGF-Primary pericyte/HUVEC in Fig. 2) showed more enhanced angiogenesis than the control Matrigel including the human primary pericyte (Primary pericyte/HUVEC in Fig. 2). In addition, no angiogenesis was observed in Matrigel including HUVEC alone (HUVEC in Fig. 2).

### Example 6: Quantitative evaluation of angiogenesis exhibited by bFGF gene-introduced human primary pericyte

Each Matrigel recovered by the procedure of Example 5 was placed in a 2 mL tube (Eppendorf, 0030120.094) and cut several times with dissecting scissors. One stainless steel bead (Qiagen, 69989) was added thereto, and 350 µL of 0.1% Brij (registered trademark) L23 solution (Sigma-Aldrich, B4184) was added. Matrigel was crushed by using TissueLyser II (Qiagen, 85300) and centrifuged under conditions of 10,000 g, 4°C, and 5 minutes, and then the supernatant was recovered. The concentration of hemoglobin in the recovered supernatant was measured by using QuantiChrom Hemoglobin Assay Kit (BioAssay Systems, DIHB-250). The method followed the product protocol. As a statistical analysis test, a t-test was carried out between two groups of the HUVEC and the control human primary pericyte (Primary pericyte/HUVEC), and the HUVEC and the bFGF gene-introduced human primary pericyte (bFGF-Primary pericyte/HUVEC).

The concentration of hemoglobin in Matrigel including bFGF gene-introduced human primary pericyte was significantly higher than that in Matrigel including the primary pericyte as the control (Fig. 3).

### Example 7 Evaluation of improvement in blood flow by bFGF gene-introduced human primary pericyte in lower limb ischemia model

300 µL of a triple mixed anesthetic solution having the same composition as that used in Example 5 was administered to the abdominal cavity of a NOG mouse. After anesthesia, body hair around the left lower limb was removed by using a depilatory cream. After that, 300 µL of an Antisedan preparation solution obtained by adding 150 uL of Antisedan (Nippon Zenyaku Kogyo Co., Ltd.) to 24.8 mL of physiological saline was subcutaneously administered to awaken the NOG mouse. The next day, the NOG mouse was again anesthetized by using the triple mixed anesthetic solution in the same manner as the previous day and placed on the back thereof under a stereoscopic microscope, and the skin of the left lower limb was incised to expose the femoral artery and vein and the saphenous artery and vein. The blood vessels branching from the femoral artery and vein were ligated, then the femoral artery and vein and the saphenous artery and vein were excised, and the skin was sutured. Subsequently, the NOG mouse was placed prone, the skin near the gastrocnemius muscle was incised, and the marginal vein was cut. The skin was sutured again, and 300 µL of the Antisedan preparation solution was subcutaneously administered to awaken the NOG mouse. At week 2 after the operation, the NOG mouse was anesthetized with the triple mixed anesthetic solution, kept warm for 10 minutes on a warming plate at 36°C, and the blood flow in the lower limb was analyzed with a blood flow imaging apparatus (moorLDI2-IR, moor instruments). The blood flow signal value of the operated ischemic limb was divided by the blood flow signal value of the non-operated normal limb to calculate the blood flow ratio (in terms of %). At the same time, the number of necrotic nails on the toes of the lower limb of the NOG mouse whose blood flow was measured was recorded. Among a plurality of lower limb ischemia model mice that were subjected to the above operation, mice that were individuals having a blood flow ratio of 20% to 40% and that had a number of necrotic nails of 4 or 5 were selected as evaluation mice, and grouped such that the average of the blood flow ratio was the same between two groups. The next day, the NOG mice grouped into two groups were anesthetized with a triple mixed anesthetic drug, and the skin of the left lower limb was incised to expose the muscle. In one group, 10 µL of 3 × 10⁶ bFGF gene-introduced human primary pericytes suspended in 100 µL of Megacell Dulbecco's Modified Eagle's Medium were administered to each of a total of 10 locations consisting of a total of 9 locations from the left lower femoral muscle to the gastrocnemius muscle and 1 location on the sole of the left lower limb (cell-administered group; 8 animals). As a control, only Megacell Dulbecco's Modified Eagle's Medium was similarly administered to the other group (medium-administered group; 7 animals). At week 2, 4, and 6 after administration of the cells or the medium, the blood flow in the lower limb was analyzed with the blood flow imaging apparatus moorLDI2-IR in the same manner as described above, and the blood flow ratio (in terms of %) (Fig. 4, left) obtained by dividing the signal value of the blood flow in the ischemic limb by the signal value of the blood flow in the normal limb and AUC (Area Under Curve) up to week 6 after cell administration were calculated (Fig. 4, right). Here, AUC was defined as the area under the polygonal line drawn when the time is plotted on the X axis and the blood flow ratio is plotted on the Y axis. A t-test was carried out as a statistical analysis test.

In the lower limb ischemia model mice, the administration of the bFGF gene-introduced human primary pericyte significantly exhibited a blood flow improving effect.

### Example 8: Induction of differentiation of ES cell into early mesodermal cell

230 µL of Matrigel Human ES Cell Qualified Matrix (Corning, 354277) is added to 25 mL of DMEM/Ham F12 medium (Nacalai Tesque, 11581-15), and 1.5 mL is added to each of 3 wells on a 6-well plate (Iwaki, 3810-006) and allowed to stand at room temperature for 3 hours to prepare a Matrigel-coated plate. A human ES cell is seeded on the Matrigel-coated plate and cultured by using STEMdiff Mesoderm induction Medium (STEMCELL Technologies, 05220) at 37°C in an atmosphere of 5% CO₂ to induce differentiation of the ES cell into an early mesodermal cell. Whether the obtained cell is differentiated into an early mesodermal cell is confirmed by flow cytometry. Specifically, CD140α and APLNR are selected as cell surface markers of an early mesodermal cell, and increase in the proportion of a CD140α(+) and APLNR(+) cell is confirmed by flow cytometry by using an antibody against each cell surface marker, to confirm that induction of differentiation of an ES cell into an early mesoderm has progressed.

### Example 9: Formation of spheroid from early mesodermal cell

The spheroid formation medium disclosed in literature (Vodyanik MA et al., Cell Stem Cell, (2010), 7: 718-729) is added to 1.7 × 10⁵ cells of the early mesodermal cell obtained in Example 8, which was then cultured on an EZSPHERE (trademark registered) dish (Iwaki, 11-0434) at 37°C in an atmosphere of 5% CO₂ and 5% O₂. When a spheroid is formed, the culture solution including the spheroid is passed through a 100 µm cell strainer to recover a spheroid having a size of 100 µm or more.

### Example 10: Induction of differentiation of spheroid into pericyte-like cell

Based on U.S. Patent US9868939, induction of differentiation of a spheroid into a pericyte-like cell is carried out. Specifically, all the spheroids recovered in Example 9 are suspended in a pericyte differentiation induction medium (see below), seeded on a dish coated with Fibronectin and Human type 1 Collagen, and cultured. When monolayer cell proliferation is observed on the bottom of the dish, the supernatant is removed, washing with PBS is carried out, then Accutase is added, and the cell is detached. The cell suspension is recovered into the dish by using the pericyte growth medium (see Example 1) and centrifuged. The supernatant is removed, the pericyte growth medium is added, seeding on a collagen-coated dish is carried out, and further culture is carried out.

### [Pericyte differentiation induction medium]

The composition is as follows:
- 50% Stemline (registered trademark) II Hematopoietic Stem Cell Expansion Medium (Sigma-Aldrich, S0192)
- 50% Human Endothelial SFM (ThermoFisher Scientific, 11111044)
- 1% GlutaMax
- 0.05% Ex-CYTE NZ Growth Enhancement Media Supplement (Merck, 81150N)
- 100 µM Monothioglycerol (FUJIFILM Wako Pure Chemical Corporation, 195-15791)
- 10ng/mL Animal-free Recombinant Human FGFbasic-TS
- 50ng/mL Recombinant Human PDGF-BB Protein (R&D Systems, 220-BB)

### Example 11: Introduction of bFGF gene into human ES cell-derived pericyte-like cell

For the human ES cell-derived pericyte-like cell prepared in Example 10, a bFGF gene is introduced into a human ES cell-derived pericyte-like cell in the same manner as for the human primary pericyte in Example 3, by using a lentivirus suspension for bFGF gene introduction prepared in the same manner as in Example 2.

### Example 12: Quantitation of bFGF expression level in bFGF gene-introduced human ES cell-derived pericyte-like cell

The bFGF expression levels in the bFGF gene-introduced human ES cell-derived pericyte-like cell prepared in Example 11 and the human ES cell-derived pericyte-like cell obtained in Example 10 as a control are quantified in the same manner as in Example 4. The bFGF gene-introduced human ES cell-derived pericyte-like cell exhibits a higher bFGF expression level than the control.

### Example 13: Evaluation of angiogenic potential of bFGF gene-introduced human ES cell-derived pericyte-like cell

Evaluation of the angiogenic potential of a bFGF gene-introduced human ES cell-derived pericyte-like cell can be carried out in the same manner as in Examples 5 and 6 in which the angiogenic potential of a bFGF gene-introduced human primary pericyte was evaluated. The bFGF gene-introduced human ES cell-derived pericyte-like cell exhibits higher angiogenic potential than the control human ES cell-derived pericyte-like cell.

### Example 14 Evaluation of improvement in blood flow by bFGF gene-introduced human ES cell-derived pericyte-like cell in lower limb ischemia model

Evaluation of improvement in blood flow in vivo by a bFGF gene-introduced human ES cell-derived pericyte-like cell can be carried out in the same manner as in Example 7 in which improvement in blood flow by a bFGF gene-introduced human primary pericyte was evaluated by using a lower limb ischemia model mouse. Administration of the bFGF gene-introduced human ES cell-derived pericyte-like cell exhibits a high blood flow improving effect in the model mouse.

### INDUSTRIAL APPLICABILITY

The pericyte introduced a bFGF gene of the present invention has excellent angiogenic action and can be used for an angiogenic therapy for critical lower limb ischemia or the like.

### SEQUENCE LISTING FREE TEXT

The nucleotide sequence shown in SEQ ID NO: 1 in the sequence listing is the gene sequence of human bFGF shown in GenBank Accession Number: M27968.1, and the amino acid sequence shown in SEQ ID NO: 2 is the amino acid sequence of human bFGF shown in GenBank Accession Number: AAA52448.1. In addition, a description of "Artificial Sequence" is provided under the number heading <223> in the sequence listing below. Specifically, the nucleotide sequence shown in SEQ ID NO: 3 in the sequence listing is a nucleotide sequence that encodes the amino acid sequence of a human bFGF analog shown in SEQ ID NO: 4. In addition, the nucleotide sequence shown in SEQ ID NO: 5 in the sequence listing is the nucleotide sequence of the human bFGF expression plasmid (pLe6-Bmp-bFGF) used in an Example of the present application.

## Claims

1. A pericyte introduced a basic fibroblast growth factor (bFGF) gene.

2. The pericyte according to claim 1, wherein the pericyte is a primary pericyte.

3. The pericyte according to claim 1, wherein the pericyte is a pericyte-like cell obtained by inducing differentiation of a pluripotent stem cell.

4. The pericyte according to claim 3, wherein the pluripotent stem cell is a human pluripotent stem cell.

5. The pericyte according to claim 3 or 4, wherein the pluripotent stem cell is an embryonic stem cell (ES cell) or an induced pluripotent stem cell (iPS cell).

6. A pharmaceutical composition for an angiogenic therapy, comprising the pericyte according to any one of claims 1 to 5.

7. The pharmaceutical composition according to claim 6, wherein the angiogenic therapy is a treatment for critical lower limb ischemia.

8. The pharmaceutical composition according to claim 6 or 7, wherein the pharmaceutical composition is used in combination with a vascular endothelial cell.

9. A pharmaceutical composition for an angiogenic therapy, comprising a combination of the pericyte according to any one of claims 1 to 5 and a vascular endothelial cell.

10. The pharmaceutical composition according to claim 9, wherein the angiogenic therapy is a treatment for critical lower limb ischemia.

11. A method for producing the pericyte according to any one of claims 1 to 5.

12. An angiogenic therapy comprising administering a therapeutically effective amount of the pericyte according to any one of claims 1 to 5 to a subject.

13. The angiogenic therapy according to claim 12, wherein the angiogenic therapy further comprises administering a vascular endothelial cell.

14. The angiogenic therapy according to claim 12 or 13, wherein the angiogenic therapy is a treatment for critical lower limb ischemia.

15. Use of the pericyte according to any one of claims 1 to 5 in production of a pharmaceutical composition for an angiogenic therapy.

16. Use of the pericyte according to any one of claims 1 to 5 in production of a pharmaceutical composition for treatment for critical lower limb ischemia.

17. The pericyte according to any one of claims 1 to 5 for use in an angiogenic therapy.

18. The pericyte according to any one of claims 1 to 5 for use in treatment for critical lower limb ischemia.
